Europäisches Patentamt

European Patent Office

Office européen des brevets

⑩

(11) Publication number: **0 117 621**
**B1**

(12) **EUROPEAN PATENT SPECIFICATION**

(45) Date of publication of patent specification: **04.04.90**

(21) Application number: **84300425.0**

(22) Date of filing: **25.01.84**

(51) Int. Cl.⁵: **A 61 K 7/32**

(54) **Cosmetic.**

(30) Priority: **02.02.83 GB 8302824**

(43) Date of publication of application:
**05.09.84 Bulletin 84/36**

(45) Publication of the grant of the patent:
**04.04.90 Bulletin 90/14**

(84) Designated Contracting States:
**AT BE CH DE FR GB IT LI LU NL SE**

(56) References cited:
**EP-A-0 018 137
DE-A-3 015 450
GB-A-1 536 222
GB-A-2 113 116
US-A-4 071 374**

(73) Proprietor: **BEECHAM GROUP PLC
Beecham House Great West Road
Brentford Middlesex TW8 9BD (GB)**

(72) Inventor: **Fairhurst, Edgar
48 Warenne Road
Fetcham Leatherhead Surrey (GB)**
Inventor: **Keyser, Anne Marion
"West Riddens" Anstye
Haywards Heath Sussex (GB)**

(74) Representative: **Russell, Brian John et al
Beecham Pharmaceuticals Great Burgh
Yew Tree Bottom Road
Epsom Surrey KT18 5XQ (GB)**

The file contains technical information
submitted after the application was filed and
not included in this specification

Courier Press, Leamington Spa, England.

## Description

The present invention relates to a new use of silanised adsorbents and to deodorant compositions containing such adsorbents.

Silanised silica gel is a known adsorbent and may be produced either by

a) treating fine particles of silica gel with a reactive silane thereby replacing many of the hydroxy groups with substituted siloxy groups, or

b) polymerising a suitable siloxane monomer in the presence of particles of silica gel thereby coating the particles with a polysiloxane.

Reactive silanes useful in process (a) above include alkyl, aryl especially phenyl and aralkyl especially benzyl halosilanes and -silazanes and alkyl, aryl especially phenyl, and aralkyl especially benzyl alkoxysilanes and -silazanes.

Particular examples of such silanes include

dimethyl monochlorosilane, dimethyl dichlorosilane, trimethylchlorosilane, hexamethyl disilazane, trichloro-octadecylsilane.

Suitable siloxane monomers for use in process (b) above include hydrogen polysiloxanes such as methyl hydrogen polysiloxane and alkyl, aryl and aralkyl cyclotetrasiloxanes, especially octamethylcyclotetrasiloxane. Polymerisation may be effected by heating or by chemical catalysis.

Similar techniques may be applied to produce silanised alumina, calcium carbonate, hydroxyapatite, iron oxide, mica, talc and derivatives thereof including titanated mica and talc. Process (a) above is suitable for adsorbents with free hydroxyl groups and process (b) may be used on most finely divided solids to produce a silanised adsorbent.

As used herein the term 'silanised adsorbents' refers to silanised silica gel.

It has now been found that silanised adsorbents have particularly advantageous properties as adsorbents of the low molecular weight compounds considered to be the major components of body odour.

Accordingly the present invention provides a method for reducing or preventing body odour which method comprises a silanised adsorbent to the body surface.

Preferably the silanised adsorbent is silanised silica gel. Suitably the silanised silica gel is silanised silica gel having mean particle size of from 0.1 to $20 \times 10^{-6}$, the 'particles' being aggregates of very fine 'primary particles' of such small size.

Preferably the silanised adsorbent is applied at least to the axillae, genital regions and/or the feet.

Whilst the silanised adsorbent may be appied directly to the body, preferably it is applied in the form of deodorant composition.

Accordingly in another aspect the present invention provides a deodorant composition comprising a silanised adsorbent and an inert carrier or diluent therefor.

Suitably the composition may be presented in conventional deodorant form, for instance as a gel, roll-on formulation, cream, gel, lotion or stick, and will contain conventional carriers and diluents known to be suitable for each particular presentation. The composition may also comprise optional accessory ingredients such as antiperspirants, perfume, colouring and preserving agents, antibacterials and antimicrobial agents such as Irgasan and chlorhexidine.

Preferably the compositions described above comprise from 0.1 to 5% w/w of silanised adsorbent, more preferably from 0.1 to 2%.

Silanised adsorbents can be incorporated in non-aqueous media without difficulty. However to achieve homogeneity in products having an aqueous base it is advantageous, though not always essential, to include at least 2% w/w or a lower alkanol, such as isopropanol or, preferably, ethanol. More preferably the product contains from 10 to 50% w/w of an alkanol.

The following Examples illustrate the invention.

As used herein P.E.G. stands for polyethylene glycol.

### Example 1
#### High alcohol roll-on antiperspirant deodorant

| a) | %w/w |
|---|---|
| Ethanol | 60.09 |
| Hydroxypropylcellulose | 0.66 |
| Aluminium chlorhydrate (50% soln) | 32 |
| Irgasan DP 300® (Triclosan) | 0.25 |
| Myristic acid | 1.5 |
| Polyethylene glycol ether of lauryl alcohol | 3.5 |
| Silanised silica gel | 2 |
| | 100 |

# EP 0 117 621 B1

### Example 1
### High alcohol roll-on antiperspirant deodorant

| b) | %w/w |
|---|---|
| Ethanol | 58.34 |
| Hydroxypropylcellulose | 0.66 |
| Polypropylene glycol ether of myristyl alcohol | 1.5 |
| Aluminium Zirconium Tetrachlorohydrate | 37.5 |
| Silanised silica gel | 2 |
| | 100 |

### Example 2
### Quick Drying Emulsion Roll-on Antiperspirant Deodorant

| a) | %w/w |
|---|---|
| Q2-3225C® (emulsifier) | 10.5 |
| Silicone DC 344® (Cyclomethicone) | 16.3 |
| Aluminium chlorhydrate (50% active soln.) | 38.2 |
| Polyglyceryl-4 Oleate | 1.5 |
| De-ionised water | 31.5 |
| Silanised silica gel | 2 |
| | 100 |

| b) | |
|---|---|
| Q2-3225C® (emulsifier) | 10.5 |
| Polyglyceryl-4 Oleate | 1.5 |
| Aluminium Zirconium Tetrachlorhydrate/glycine complex | 42.8 |
| De-ionised water | 29 |
| Silicone DC 344® (Cyclomethicone) | 14.2 |
| Silanised silica gel | 2 |
| | 100 |

### Example 3
### Stick antiperspirant Deodorants
### Suspension type

| a) | %w/w |
|---|---|
| Stearyl alcohol | 24 |
| Mineral Oil | 25 |
| Silicone DC 345 ® (cyclomethicone) | 17 |
| Aluminium chlorhydrate (ultrafine) | 25 |
| Octyl Hydroxystearate | 8 |
| Silanised silica gel | 1 |
| | 100 |

| b) | |
|---|---|
| Stearyl alcohol | 25 |
| Mineral Oil | 28.5 |
| Octyl Hydroxystearate | 8 |
| Silanised silica gel | 0.5 |
| Aluminium zirconium tetrachlorhydrate | 20 |
| Silicone DC 345 ® (cyclomethicone) | 18 |
| | 100 |

| c) Alcohol type | |
|---|---|
| Ethanol | 23 |
| Silicone DC 344 ® (cyclomethicone) | 10 |
| Talc | 20 |
| PEG—200 Trihydroxystearin | 5 |
| Aluminium chlorhydrate (ultrafine) | 20 |
| Silanised silica gel | 2 |
| Stearyl alcohol | 20 |
| | 100 |

3

## EP 0 117 621 B1

| d) | %w/w |
|---|---|
| Ethanol | 23 |
| Silicone DC 344 ® (cyclomethicone) | 10 |
| Talc | 20 |
| PEG—200 Trihydroxy stearin | 5 |
| Aluminium zirconium tetrachlorhydrate | 20 |
| Silanised silica gel | 2 |
| Stearyl alcohol | 20 |
| | 100 |

## Claims

1. A method for reducing or preventing body odour which method comprises applying silanised silica gel to the body surface.

2. A method as claimed in claim 1 wherein the silanised silica gel is applied in the form of a deodorant composition.

3. A deodorant composition comprising silanised silica gel and an inert diluent or carrier therefor.

4. A composition as claimed in claim 3 comprising at least 2% w/w of the total composition of ethanol.

## Patentansprüche

1. Eine Methode zur Verringerung oder Verhinderung von Körpergeruch, welche Methode das Aufbringen von silanisiertem Siliciumdioxidgel auf die Körperoberfläche umfaßt.

2. Eine Methode in Anspruch 1 beansprucht, in welcher das silanisierte Siliciumdioxidgel in Form einer desodorierenden Zusammensetzung aufgebracht wird.

3. Eine desodorierende Zusammensetzung, umfassend silanisiertes Siliciumdioxidgel und ein inertes Verdünnungsmittel oder einen Träger für dieses.

4. Eine Zusammensetzung wie in Anspruch 3 beansprucht, umfassend mindestens 2 Gewichtsprozent, bezogen auf die gesamte Zusammensetzung, an Äthanol.

## Revendications

1. Méthode pour réduire ou empêcher une odeur corporelle, caractérisée en ce qu'elle comprend l'application d'un gel de silica silanisé sur la surface du corps.

2. Méthode suivant la revendication 1, caractérisée en ce que le gel de silice silanisé est appliqué sous la forme d'une composition déodorante.

3. Composition déodorante, caractérisée en ce qu'elle comprend un gel de silice silanisé et un diluant ou un support inerte pour celui-ci.

4. Composition suivant la revendication 3, caractérisée en ce qu'elle comprend au moins 2% en poids d'éthanol par rapport au poids de la composition totale.

4